# EUROPEAN PATENT SPECIFICATION

(11) **EP 3 071 968 B1**
(45) Date of publication and mention of the grant of the patent: **22.04.2020**
(21) Application number: 14783894.0
(22) Date of filing: 24.09.2014
(51) Int. Cl.: G01N 33/542, G01N 33/58, G01N 21/64

(54) **METHOD OF ANALYSIS**
ANALYSEVERFAHREN
PROCÉDÉ D'ANALYSE

(30) Priority: 19.11.2013 FI 20130344; 19.11.2013 FI 20130342; 19.11.2013 FI 20130345
(43) Date of publication of application: 28.09.2016
(73) Proprietor: Kemira Oyj, 00180 Helsinki (FI)
(72) Inventor: HÄRMÄ, Harri, FI-20520 Turku (FI); LEHMUSTO, Mirva, FI-20380 Turku (FI); TIITTANEN, Satu, FI-20750 Turku (FI); SIIVONEN, Joonas, FI-20540 Turku (FI); VÄISÄNEN, Pave, FI-00720 Helsinki (FI); MUNDILL, Paul, FI-02210 Espoo (FI)
(74) Representative: Berggren Oy, Helsinki & Oulu
(86) International application number: PCT/FI2014/050728
(87) International publication number: WO 2015/075300

(56) References cited:
- WO-A2-2011/088193
- US-A1- 2013 171 623
- US-A1- 2013 171 624
- GILLES MULLER: "Luminescent chiral lanthanide(iii) complexes as potential molecular probes", DALTON TRANSACTIONS, no. 44, 1 January 2009 (2009-01-01), page 9692, XP055159688, ISSN: 1477-9226, DOI: 10.1039/b909430j
- BÜNZLI JEAN-CLAUDE G ET AL: "Taking advantage of luminescent lanthanide ions", CHEMICAL SOCIETY REVIEWS, CHEMICAL SOCIETY, LONDON, GB, vol. 34, no. 12, 1 December 2005 (2005-12-01), pages 1048-1077, XP002593476, ISSN: 0306-0012, DOI: 10.1039/B406082M [retrieved on 2005-09-20]
- KARHUNEN ULLA ET AL: "Luminescence switching by hybridization-directed mixed lanthanide complex formation", ANALYTICAL CHEMISTRY, AMERICAN CHEMICAL SOCIETY, US, vol. 82, no. 2, 15 January 2010 (2010-01-15), pages 751-754, XP002593475, ISSN: 0003-2700, DOI: 10.1021/AC9020825 [retrieved on 2009-12-17]
- KARHUNEN ULLA ET AL: "Quantitative detection of well-based DNA array using switchable lanthanide luminescence", ANALYTICA CHIMICA ACTA, ELSEVIER, AMSTERDAM, NL, vol. 772, 28 February 2013 (2013-02-28), pages 87-92, XP028526740, ISSN: 0003-2670, DOI: 10.1016/J.ACA.2013.02.029
- BEKIARI V ET AL: "INTENSELY LUMINESCENT MATERIALS OBTAINED BY COMBINING LANTHANIDE IONS, 2.2'-BIPYRIDINE, AND POLY(ETHYLENE GLYCOL) IN VARIOUS FLUID OR SOLID ENVIRONMENTS", CHEMISTRY OF MATERIALS, AMERICAN CHEMICAL SOCIETY, US, vol. 11, no. 11, 1 November 1999 (1999-11-01), pages 3189-3195, XP000866820, ISSN: 0897-4756, DOI: 10.1021/CM991059B
- ARI LEHMUSVUORI ET AL: "High-performance closed-tube PCR based on switchable luminescence probes", ANALYTICA CHIMICA ACTA, ELSEVIER, AMSTERDAM, NL, vol. 731, 20 April 2012 (2012-04-20), pages 88-92, XP028509845, ISSN: 0003-2670, DOI: 10.1016/J.ACA.2012.04.027 [retrieved on 2012-05-01]

## Description

### FIELD

This invention relates to a method for examining samples including two or more organic molecules, wherein at least one is an organic polymer including two or more chelating groups, with the aid of lanthanide(III) ions.

### BACKGROUND

There exist numerous methods for quantitative analysis of organic molecules. Exemplary widely used methods are chromatography and spectroscopy. Several of the methods exploit the inherited properties of the organic molecules, such as their fluorescence, absorptivity and chirality for detection, while in some other methods the analysis is performed in the aid marker molecules which interact with the molecule to be analyzed giving rise to a specific signal which is detected. Representative marker molecules are organic dyes and luminescent lanthanide chelates. For example, US 4,565,670 discloses a heterogeneous method for fluorescence spectroscopic determination of a biologically active substance wherein a lanthanide ion is separated from EDTA-labeled immune reactant at low pH-value in solution containing a suitable detergent, a synergistic compound and a β-diketone to amplify the fluorescence after the separation. The use of luminescent stable lanthanide(III) chelates has extended the use of time resolution to homogeneous assays. For example, EP 0324 323A discloses an immunochemical assay including a luminescent lanthanide(III) chelate covalently bound to a immune reactant and one or more fluorescence-modulating substances such as proteins and detergents.

US 6,329,205 discloses visualizing of amine-containing polymers using luminescent complexes of europium(III).

Hänninen et al (J.Am. Chem. Soc. 2013, 135, pp. 7422-25) discloses an approach to fingerprinting liquids that is based on nonspecific interactions of the sample liquid, a long lifetime luminescence europium label, and various surface modulators in an array form.

In the spectroscopic assays described above the detection is based on time-resolved fluorometric analysis of signal derived from a lanthanide(III) chelate including one or more aromatic structures, which absorb the excitation energy and transfer it to the lanthanide(III) ion and chelating groups such as β-diketones and carboxylic acids. The aromatic structures exhibiting the properties described above are also known in art as antennas.

It is known that lanthanide(III) ions can be detected also in the in the absence of antenna molecules. Bekiari et al. have shown that association of lanthanide ions with the ether oxygens of the ethylene glycol groups of PEG chains results in increasing their luminescence intensity in several fluid environments (Chem. Mater. 1999, 11, 3189-3195). The document teaches that such a concentration dependency is achieved for low molecular-weight non-ionic PEG-200 and PEG-400 when measured in the presence of relatively high concentration of lanthanide ion (40 mM) and using time-resolved luminescence detection method. The lowest detectable concentration was approximately 10% (by weight) solution for PEG-200 corresponding to a 0.5 M solution.

It is known in the art that heavy metals are strong quenchers of lanthanide luminescence and that Cu²⁺ is the most efficient quencher. This phenomenon has been utilized in development of sensors for metal ions e.g. for detection of toxic metals in drinking water [Angew. Chem. Int. Ed. (2012) 51, 3532]. Otherwise, the high sensitivity to copper and other metal ions is a disadvantage whenever luminescent lanthanide chelates are used as labels in various applications. Since environmental, industrial and biological samples may include various amounts of heavy metals, gross analytical errors may result from quenching by heavy metals, especially copper.

It is obvious for a person skilled in art that the method of choice is dependent on the nature of the organic molecules to be analyzed, required detection sensitivity and so on. Although the field of analytical chemistry is well developed, there is still a need of alternative methods of analyses.

### SUMMARY

According to one aspect, of the disclosure the present technology concerns a method for examining a sample containing two or more sample substances comprising one or more organic polymers, wherein at least one organic polymer comprises two or more chelating groups selected from carboxylates, amines, sulfonates, carboxamides, phosphates and phosphonates, the method comprising two or more sub-methods for detecting signals, wherein the sub-methods are selected from fluorescence measurement, luminescence measurement, time-resolved luminescence measurement, and absorbance measurement, wherein at least one of the sub-methods comprises a time resolved luminescence measurement comprising
(i) admixing the sample and lanthanide(III) ion, wherein the total concentration of the sample substances in the sample is below 10% by weight and the concentration of the lanthanide(III) ion is below ≤ 10 mM, preferably from about 10 mM to about 0.001 mM,
(ii) detecting signal of the lanthanide(III) ion, wherein the lanthanide(III) ion is admixed in form of lanthanide(III) salt with time-gated luminescence measurement, and
(iii) calculating the quantity of one or more sample substances using the signal obtainable by the sub-method,
and calculating the quantity of the two or more sample substances based on the signals obtainable by the two or more sub-methods.

According to another aspect of the disclosure the present technology concerns a computer program product including computer executable instructions for controlling a programmable processor to quantify the two or more sample substances present in the sample, wherein the program is adapted to evaluate the data obtainable by a method according to any of claims 1-11.

Further aspects of the technology are disclosed in dependent claims.

Various exemplifying and non-limiting aspects of the disclosure both as to constructions and to methods of operation, together with additional objects and advantages thereof, will be best understood from the following description of specific exemplifying embodiments when read in connection with the accompanying drawings.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### BRIEF DESCRIPTION OF DRAWINGS

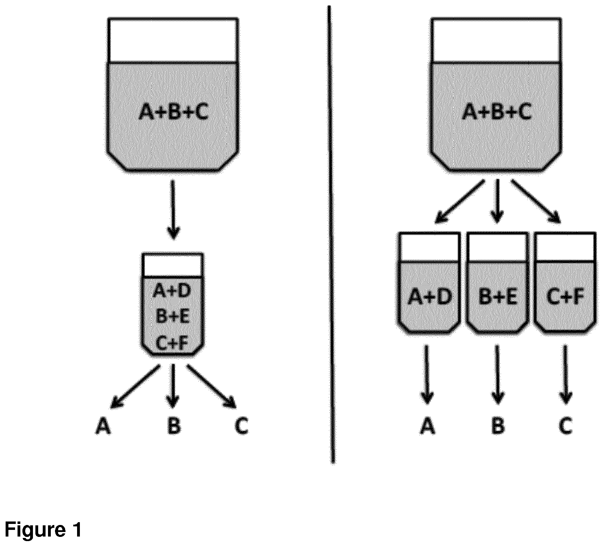

Figure 1 illustrates an exemplary schematic principle of the present technology without purification. A sample containing three organic molecules (A,B,C) to be measured with three detection chemistries (D,E,F) and individual compounds are quantified using an algorithm (bottom). The method is performed in a single vessel (left) or individual vessels (right).

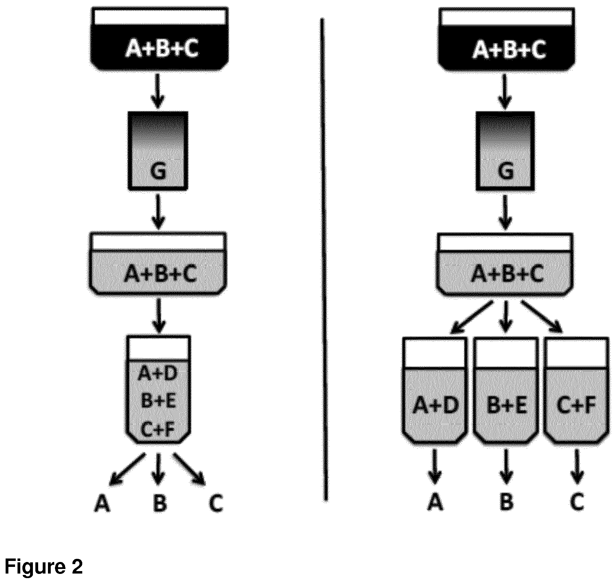

Figure 2 illustrates an exemplary schematic principle of the present technology using purification. A sample containing three organic molecules (A,B,C) to be measured is purified (G) prior to measuring with three detection chemistries (D,E,F) and individual compounds are quantified using an algorithm (bottom). The method is performed in a single vessel (left) or individual vessels (right).

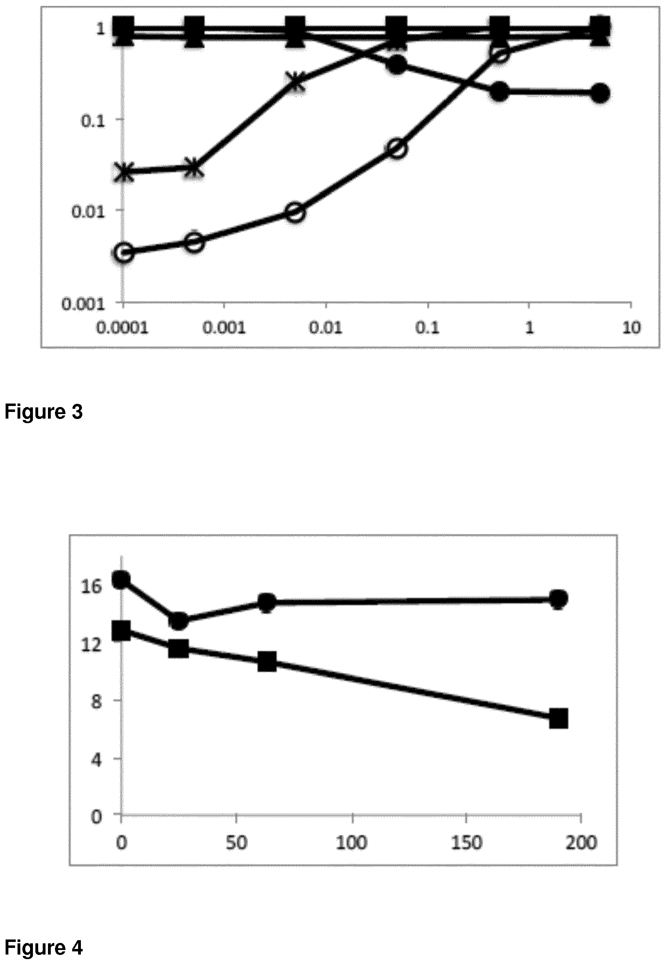

Figure 3 illustrates measurement of filled circle: polystyrene sulfonate; triangle: PEG 400; square: PEG 6000; star: polyethylene imine; open circle: BSA. Luminescence signal of Eu³⁺ was monitored at the excitation wavelength of 340 nm and emission wavelength of 615 nm. X-axis: concentration of different compounds (weight-%); Y-axis: time-resolved luminescence signal (counts).
Figure 4 illustrates measurement of BSA protein in the presence of a constant concentration of EDTA (5 mM) in H₂O. Luminescence signal of Eu³⁺ was monitored at the excitation wavelength of 285 nm (square) and 319 nm (circle) and emission wavelength of 615 nm. X-axis: BSA concentration (mM); Y-axis: time-resolved luminescence signal (counts).

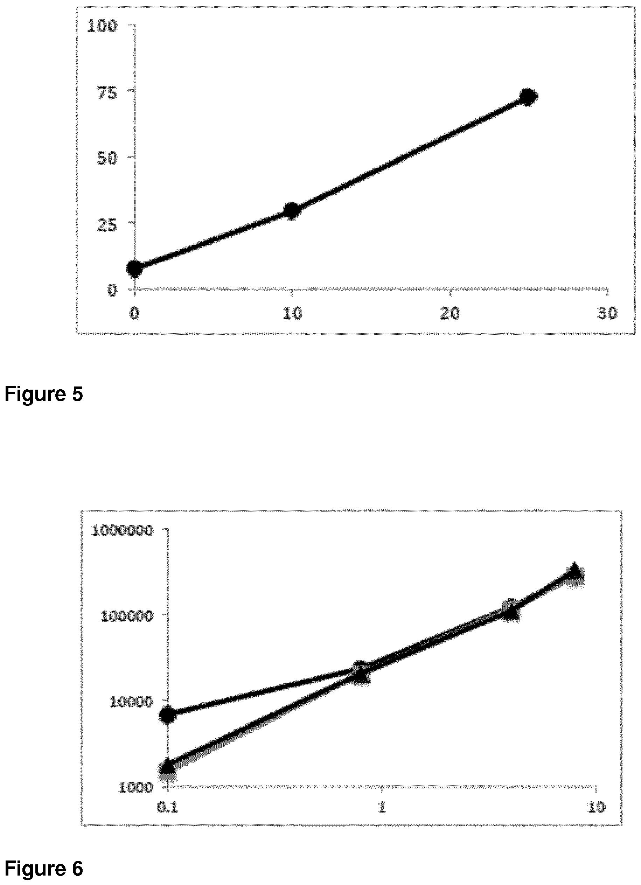

Figure 5 illustrates measurement of EDTA in the presence constant concentration of BSA (50 µM) in H₂O. Luminescence signal of Eu³⁺ was monitored at the excitation wavelength of 319 nm and emission wavelength of 615 nm. X-axis: concentration of EDTA (mM); Y-axis: time-resolved luminescence signal (counts).
Figure 6 illustrates measurement of BSA in the presence of a constant concentration of EDTA (50 µM). Luminescence signal of Eu³⁺ was monitored at the excitation wavelength of 340 nm and emission wavelength of 615 nm. Circle: BSA and EDTA in water; triangle: BSA in water; grey square: BSA and EDTA in water centrifuged Amicon Ultra-4, cut-off 30kDa wherefrom non-permeate fraction measured. X-axis: BSA concentration (µM); Y-axis: time-resolved luminescence signal (counts).

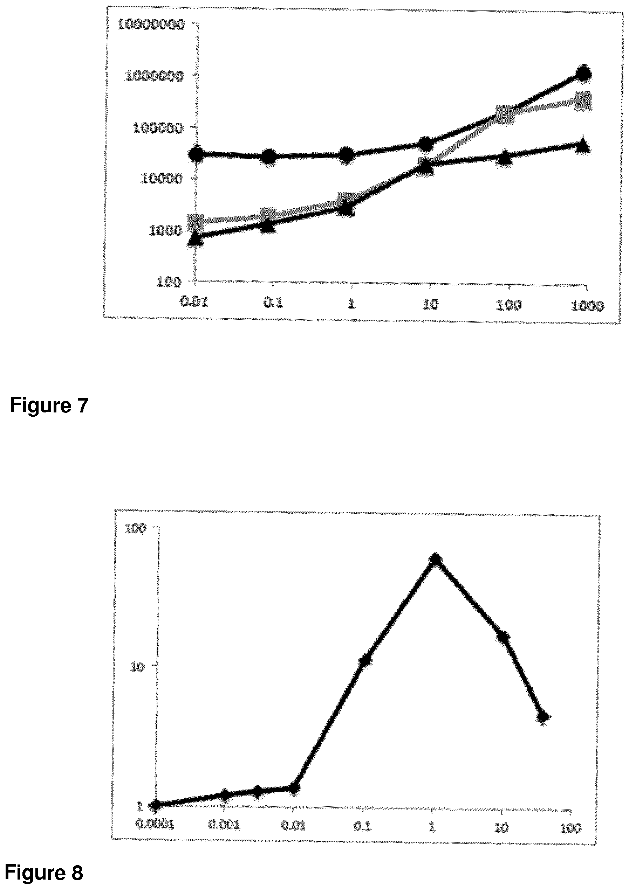

Figure 7 illustrates measurement of BSA in the presence of three different Eu³⁺ concentrations. Luminescence signal of Eu³⁺ was monitored at the excitation wavelength of 340 nm and emission wavelength of 615 nm. Circle: EuCl₃ 10 mM; Grey square: EuCl₃ 1 mM; and triangle: EuCI3 0.10 mM. X-axis BSA concentration (µM); Y-axis: time-resolved luminescence signal (counts).
Figure 8 illustrates measurement of DNA (Mr = 11000) in the presence of different Eu³⁺ concentrations. Luminescence signal of Eu³⁺ was monitored at the excitation wavelength of 340 nm and emission wavelength of 615 nm. X-axis EuCl₃ concentration (µM); Y-axis: signal-to-background ratio (S/B).

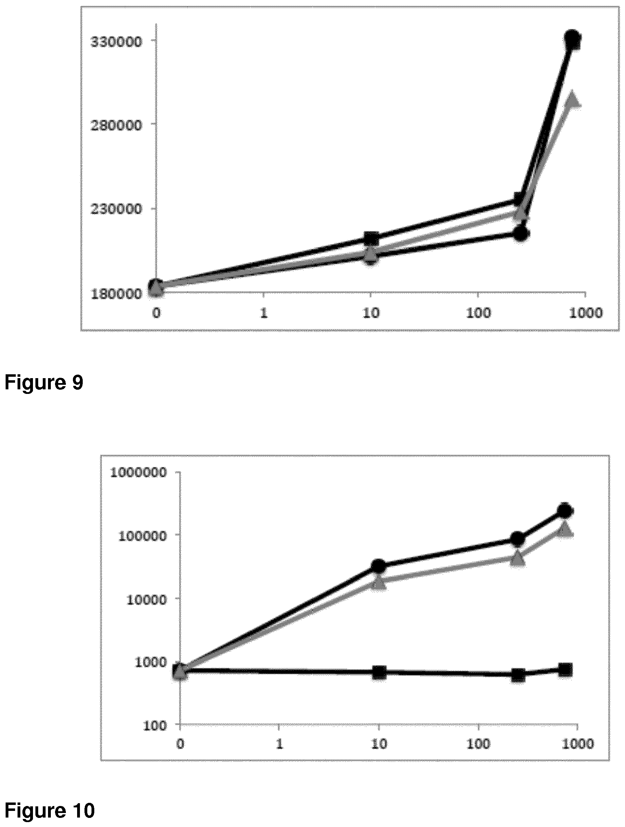

Figure 9 illustrates measurement of DNA (Mr = 11000) labeled with Eu chelate (circle), non-labeled DNA (the same strand) without any label (square) and 50% labeled DNA + 50% non-labeled DNA (triangle, grey) using terbium ion. Luminescence signal of Tb³⁺ was monitored at the excitation wavelength of 340 nm and emission wavelength of 545 nm.The total concentration of DNA was measured (labeled DNA + non-labeled DNA). X-axis: DNA concentration (nM); Y-axis: time-resolved luminescence signal (counts).
Figure 10 illustrates measurement of DNA (Mr = 11000) labeled with Eu chelate (circle), non-labeled DNA (the same strand) without any label (square) and 50% labeled DNA + 50% non-labeled DNA (triangle, grey). Luminescence signal of the Eu chelate was monitored at the excitation wavelength of 340 nm and emission wavelength of 615 nm. X-axis: DNA concentration (nM); Y-axis: time-resolved luminescence signal (counts).

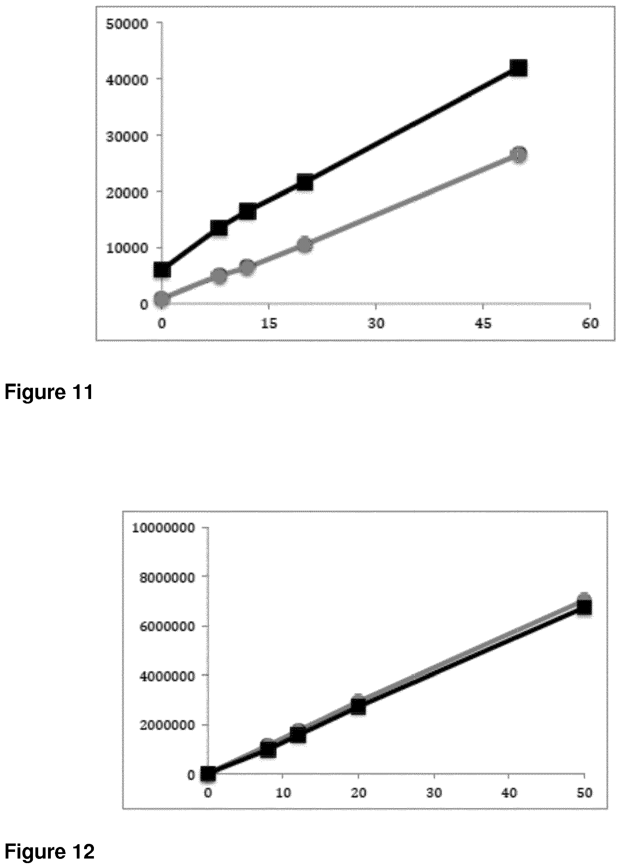

Figure 11 illustrates measurement of BSA labeled with FITC (circle, grey) and BSA labeled with FITC in the presence of 2 µM of DNA (Mr = 11000) (square) using europium ion. Luminescence signal of Eu³⁺ was monitored at the excitation wavelength of 340 nm and emission wavelength of 615 nm. The total concentration of BSA-FITC and DNA was measured. X-axis: BSA-FITC concentration (µM); Y-axis: time resolved luminescence signal (counts).
Figure 12 illustrates measurement of BSA labeled with FITC (circle, grey) and BSA labeled with FITC in the presence of 2 µM of DNA (Mr = 11000) (square) using fluorescein fluorescence detection. Luminescence signal of fluorescein was monitored at the excitation wavelength of 485 nm and emission wavelength of 535 nm. Concentration of BSA-FITC is measured. X-axis: BSA-FITC (mM); Y-axis: time-resolved luminescence signal (counts).

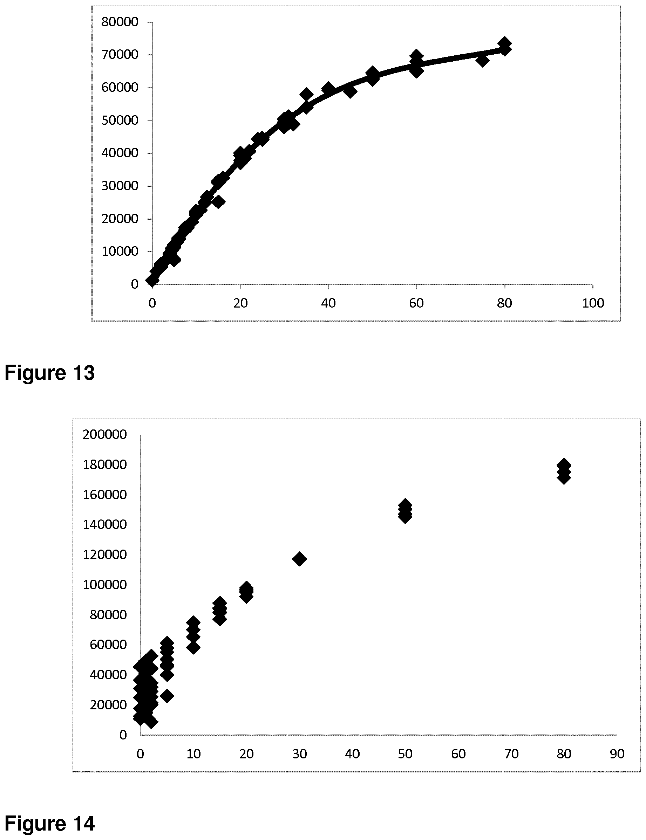

Figure 13 illustrates measurement of total concentration of a mixture of polymers P1 and P2. X-axis: total concentration of polymer in sample (ppm). Y-axis: time-resolved luminescence signal (counts).
Figure 14 illustrates the raw data with a standard curve for the measurement of a concentration of a tagged polymer P2. X-axis: Concentration of tagged polymer P2 in sample (ppm). Y-axis: time-resolved luminescence signal (counts).

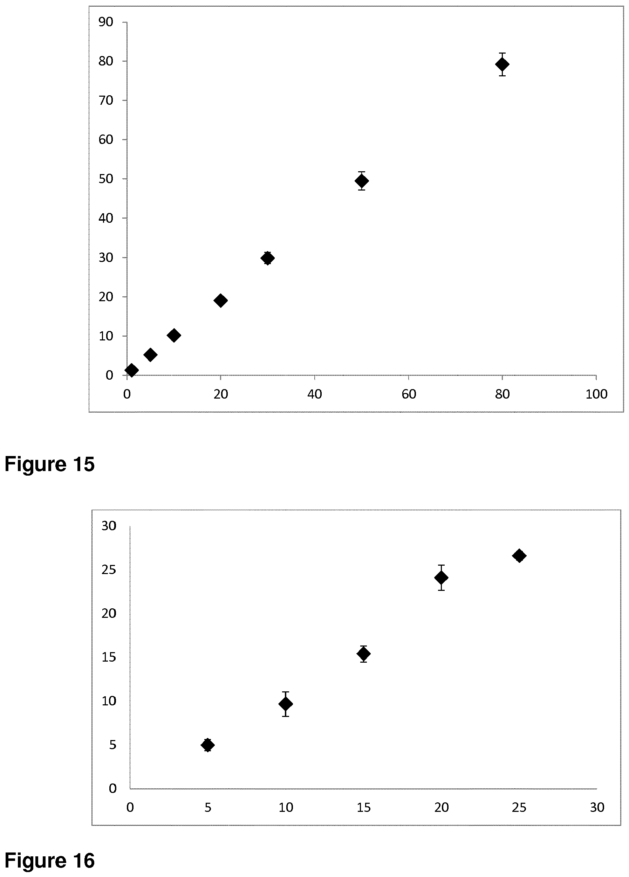

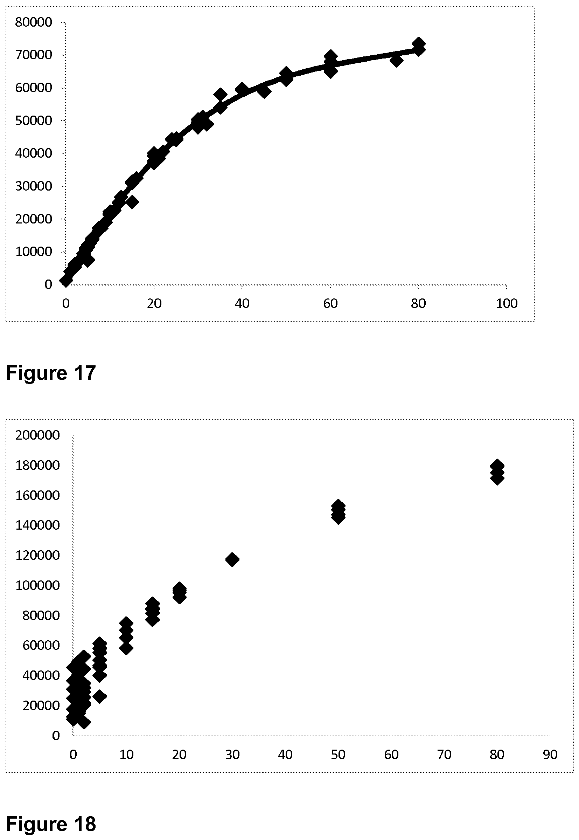

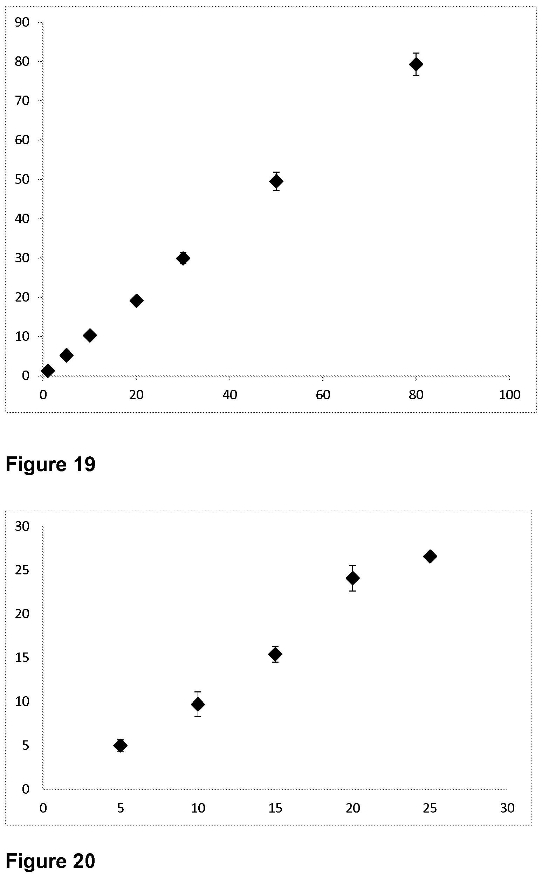

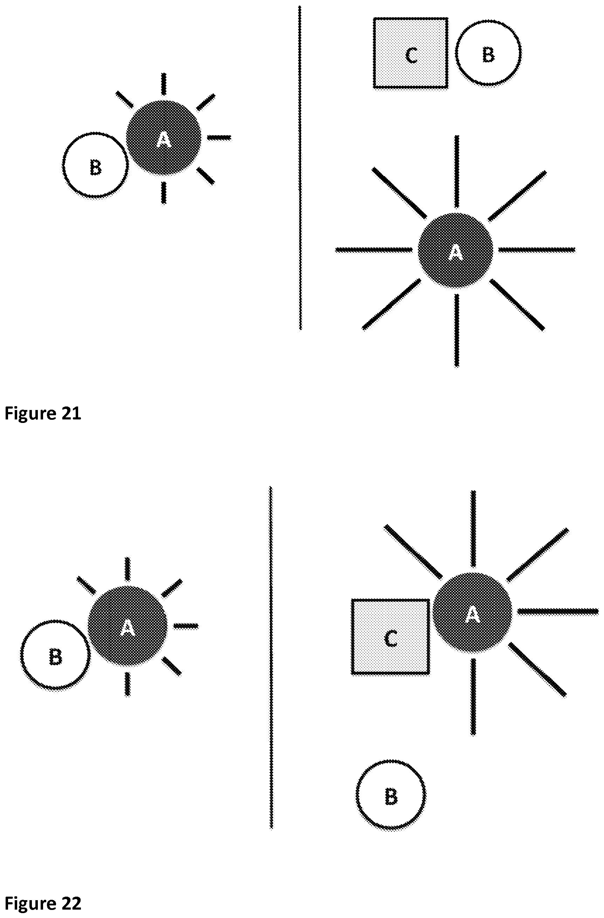

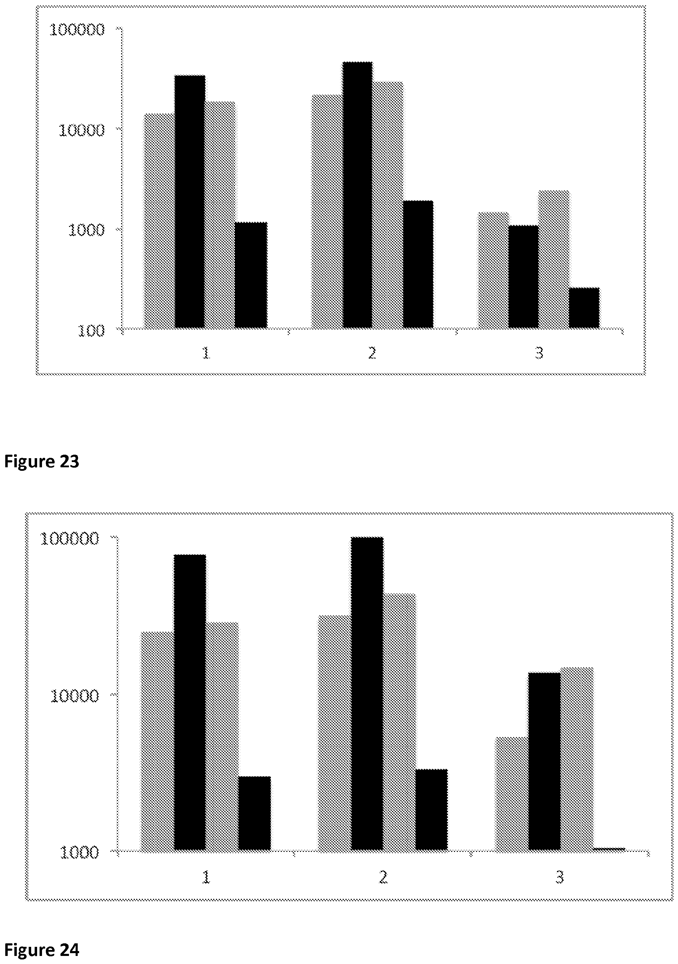

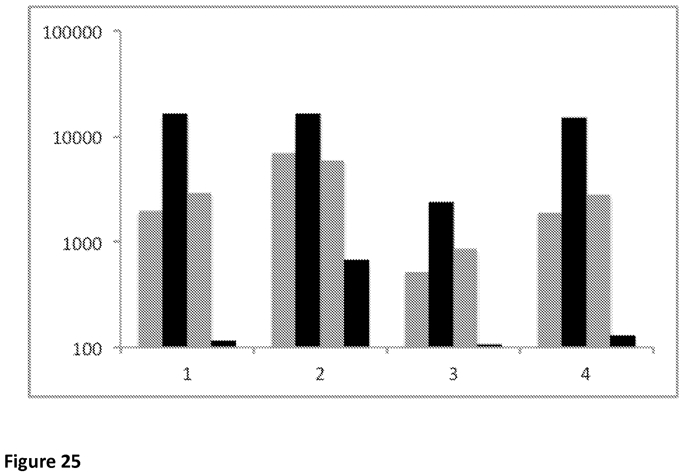

Figure 15 illustrates the prediction accuracy of P2 concentration from a validation set. X-axis: Concentration of P2 in sample (ppm). Y-axis: predicted concentration of P2 (ppm) with error bars (One standard deviation).
Figure 16 illustrates the prediction accuracy of P1 concentration from the validation set X-axis: Concentration of P1 in sample (ppm). Y-axis: predicted concentration of P1 (ppm) with error bars (one standard deviation).

### DESCRIPTION

As defined the lanthanide ion used in the at least one of the sub methods is introduced in the form of lanthanide salt, such as lanthanide(III) chloride or lanthanide(III) acetate. A preferable lanthanide(III) salt is lanthanide(III) halide, such as europium(III) chloride, terbium(III) chloride, samarium(III) chloride and dysprosium(III) chloride. Preferable lanthanide(III) chlorides are europium(III) chloride and terbium(III) chloride. An exemplary lanthanide(III) salt is europium(III) chloride. Accordingly, the lanthanide(III) ion is introduced as a salt, such as europium chloride and the lanthanide(III) chelate is formed upon conjugation of the lanthanide(III) ion with the sample.

As defined herein the term chelating group include ionizing groups. According to a preferable embodiment the chelating groups are negatively charged.

As defined herein a lanthanide chelate is a chemical compound in a form of a heterocyclic ring containing a lanthanide ion attached by coordinate bonds to at least two non-metal ions.

As defined herein, a polymer is a chemical compound or mixture of compounds consisting of repeating structural units created through a process of polymerization.

The organic molecules including two or more chelating groups to be detected according to the present technology do not need to contain aromatic groups that are able to chelate with the lanthanide ion and absorb excitation energy and transfer it essentially to lanthanide(III) ion, i.e. to act as an antenna group. The antenna group has to have sufficient energy transfer properties and it has to be in close proximity to the chelated lanthanide(III) ion.

According to one disclosure the organic polymer to be analyzed according to the present technology does not include an aromatic group wherein there are less than three bonds between the aromatic group and at least one of the two or more chelating/ionizable groups. An exemplary organic molecule wherein there is at least three bonds between at least one of the two or more ionizable groups is DNA, part of which is shown below.

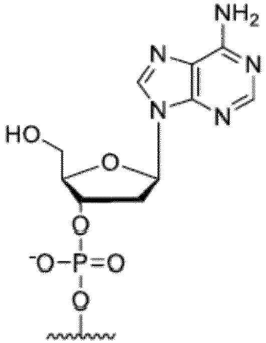

According to one embodiment the organic polymer measured according to the present technology does not include any aromatic groups.

As defined herein, the concentration of the sample substances in the sample is unknown. Prior art knows methods where the concentration of molecules in a reaction is known but the sample molecule may take two or more forms e.g. by phosphorylation/dephosphorylation on enzyme activity. In the context of this invention, the concentration of the sample substances, e.g. organic polymer is unknown and adaptation of two or more forms is not considered to be a sample of an unknown origin.

The lanthanide ion used in the present technology is selected from europium, terbium, samarium and dysprosium, preferably europium and terbium, even more preferably europium. A combination of lanthanide ions may be used according to the invention.

At least one of the sample substances is an organic polymer. According to one embodiment the its molecular weight is over 400 g/mol. Exemplary polymeric chelating molecules are DNA, polystyrene sulfonates and polyethylene imines. When charged, the organic polymer can be anionic, cationic and zwitterionic. According to one embodiment the organic polymer is not protein or oligopeptide containing serine, tyrosine or threonine residues or phosphorylated derivatives thereof.

The organic polymer measured/quantified by the method of the present technology must have at least two ionizable/chelating groups, preferably at least three ionizable/chelating groups, more preferably more than four ionizable/chelating groups and most preferably more than 12 ionizable/chelating groups. These ionizing/chelating groups are essential since they are capable to chelate with lanthanide(III) ions. The chelating groups are selected from the following: carboxylates, sulfonates, phosphates, phosphonates, carboxamides and amines, more preferably from carboxylates, sulfonates, phosphates and amines, most preferably from carboxylates and sulfonates. The amines can be primary, secondary or tertiary, and the phosphates can be primary or secondary.

Accordingly, the chelating groups are the following
- carboxylate (COO⁻),
- sulfonate (-SO₃⁻)
- carboxamide (-CONH-,-NHCO-, -CONH₂)
- phosphate 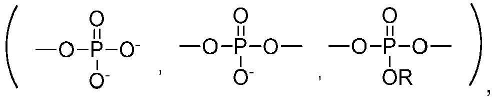 wherein R is alkyl
- phosphonate 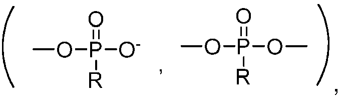 wherein R is alkyl and amine

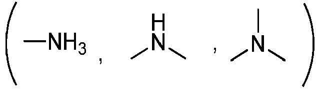

The net charge of the organic polymer may change as a function of pH.

According to one disclosure the non-polymeric organic molecules quantified by the method of the present technology has at least two ionizable/chelating groups, preferably at least three ionizable groups, more preferably more than four ionizable groups. The ionisable groups are selected from the following: carboxylates, sulfonates, phosphates, phosphonates, carboxamines and amines, more preferably from carboxylates, sulfonates, phosphates and amines, most preferably from carboxylates and sulfonates. The amines can be primary, secondary or tertiary, and the phosphates can be primary or secondary.

Accordingly, the chelating groups are the following
- carboxylate (COO⁻),
- sulfonate (-SO₃⁻)
- carboxamide (-CONH-,-NHCO-, -CONH₂)
- phosphate 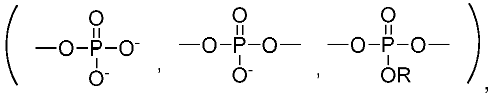 wherein R is alkyl
- phosphonate 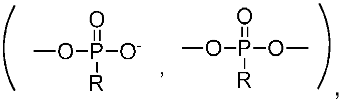 wherein R is alkyl and amine

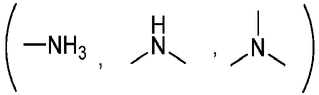

According to one preferable disclosure the sample substances measured according to the present technology include two or more chelating/ionizing groups. The sample includes at least one organic polymer which is detected using the photophysical properties of lanthanide(III) ions. The lanthanide ion is selected from europium, terbium, samarium and dysprosium, preferably europium and terbium, more preferably europium. A combination of different lanthanide ions may be used according to the invention.

Figure 3 illustrates an exemplary embodiment of the present technology. Different compounds were measured in the presence of 1 mM Eu³⁺. All data were monitored at the excitation wavelength of 340 nm and emission wavelength of 615 nm, and the europium signal was detected as a function of the concentration of the organic polymer. It is clearly seen that the europium signal is dependent on the concentration of zwitter ionic (open circle; BSA), anionic (circle; polystyrene sulfonate), and cationic (star; polyethylimine) organic molecules. In strict contrast the europium signal does not change as a function of non-charged organic molecules such as PEG 400, (square) and PEG 6000 and (triangle).

Figure 4 illustrates another exemplary embodiment of the present technology wherein BSA protein was measured in the presence of a constant concentration of EDTA (5 mM). BSA was monitored at the excitation wavelength of 285 nm and emission wavelength of 615 nm as a function of BSA concentration (squares). The constant concentration of EDTA gives a constant response (circles) at the excitation wavelength of 319 nm and emission wavelength of 615 nm without any major disturbance of varying BSA concentration. Accordingly, one organic polymer can be detected and quantified in the presence of another charged organic molecule.

Figure 5 illustrates another exemplary embodiment of the present technology wherein EDTA was measured in the presence of a constant concentration of BSA. EDTA was monitored at the excitation wavelength of 319 nm and emission wavelength of 615 nm without any major disturbance of varying the concentration of the other BSA concentration. Accordingly, one molecule can be detected and quantified in the presence of another molecule.

In the examples shown in Figures 4 and 5 the difference of the first excitation wavelength and the second excitation wavelength is 34 nm. Substantially identical results were obtained when the difference was limited to 20 nm.

According to one embodiment the present technology includes a method for examining a sample containing first sample substance and a second sample substance, the method comprising
- admixing the sample and lanthanide(III) ion,
- excitating the sample at the first wavelength, and detecting the signal derived from the lanthanide(III) ion with time-gated luminescence measurement,
- excitating the sample at the second wavelength, and detecting the signal derived from the lanthanide(III) ion with time-gated luminescence measurement, and
- measuring the presence quantity of the first sample substance and the second sample substance based on the signal of the lanthanide(III) ion,
wherein the sample substance is preferably an organic polymer.

The strongest lanthanide emission signal is obtainable when the excitation wavelength is substantially at the excitation maximum of sample substances. Accordingly the first excitation wavelength is substantially the excitation maximum of the first sample substance in the presence of the lanthanide ion, and the second excitation wavelength is substantially the excitation maximum of the second sample substance in the presence of the lanthanide ion. Alternatively, a common excitation wavelength can be used to quantify the concentration of all sample substances and a second excitation wavelength to quantify the second sample substance. Thereafter, an algorithm is used to calculate the quantity of the first sample substance. According to one embodiment the excitation spectra of the sample substances are measured prior to performing the method of the present technology. Alternatively, they can be obtained or estimated from literature. According to one embodiment the difference in the first excitation wavelength and the second excitation wavelength is more than 10 nm, preferably more than 20 nm, most preferably more than 50 nm. It is known for a person skilled in art that the more the excitation spectra of the first sample substance and the second sample substance overlap, the more the more the method may be disturbed. Alternatively, multiple emission wavelengths may be selected to quantify the content of the sample.

According to another disclosure the sample includes three or more sample substances, e.g. organic polymers, and the excitating is performed at three or more wavelengths chosen according to the properties of the sample substances.

Figure 6 illustrates another exemplary embodiment of the present technology wherein BSA was measured in the presence or absence of a constant concentration of EDTA (50 µM). All data was monitored at the excitation wavelength of 340 nm and emission wavelength of 615 nm. It is clearly seen that the presence of EDTA disturbs the measurement of BSA and purification removes this effect. Purification step does not have effect on the BSA detection. The purification, e.g. by centrifugation prior to admixing with lanthanide(III) ion may be necessary when the sample contains insoluble particles or other components which need to be removed prior to analysis.

Figure 7 and 8 illustrate another exemplary embodiment of the present technology using varying concentration of Eu³⁺. In Figure 7 BSA was measured in the presence of three different concentrations of Eu³⁺. In Figure 8 DNA was measured in the presence of different concentrations of Eu³⁺. All data was monitored at the excitation wavelength of 340 nm and emission wavelength of 615 nm. It is seen that the method allows detection of low amounts of charged organic molecules even with 0.001 mM concentration of lanthanide(III) ion.

Figures 9 and 10 illustrate another exemplary embodiment of the present technology. Figure 10 illustrates measurement of DNA labeled with a luminescent Eu chelate, measurement of the same DNA strand without any label and measurement of a 1:1 mixture of labeled and non-labeled DNA sequence. Since the measurement is based on the chelate luminescence, only the labeled sequences can be detected. Figure 9 illustrates an exemplary embodiment of the present technology wherein the same sequences were measured in the presence of terbium ion (1mM), and the detection was performed using terbium window. Accordingly, total DNA concentration can be measured independently on the level of DNA labeling. Accordingly, the present technology allows measurement of total amount charged organic molecules in a sample (here DNA) and the measurement of labeled charged organic molecules in the sample (here DNA).

Figure 11 illustrates another exemplary embodiment of the present technology wherein BSA labeled with FITC was measured in the absence and presence of 1 mM DNA using europium ion parameters (luminescence). Figure 12 illustrates another exemplary embodiment of the present technology wherein BSA labeled with FITC was measured in the absence and presence of 1 mM DNA using FITC parameters (fluorescence). Accordingly, a charged organic molecule (here BSA) can be measured quantitatively in the presence of another charged organic molecule (here DNA) since DNA does not disturb FITC signal. Accordingly, two charged organic molecule (here BSA and DNA) can be measured quantitatively in the presence of one another using a mathematical algorithm when BSA is measured using FITC parameters and total concentration of BSA and DNA is measured using europium ion parameters.

The method for examining a sample containing two or more sample substances according to the present technology is described in the following non-restrictive example.

A sample includes three sample substances, i.e. A, B and C. A is an organic polymer including two or more chelating groups. Compounds A and B do not have chromophores but C does. The sample is divided into three wells. For each well a method of analysis called a sub-method is performed.

### The first well:

(i) the sample and lanthanide(III) are mixed. The total concentration of A, B and C is kept below 0.5 M and the concentration of the lanthanide(III) ion is from about 10 mM to about 0.001 mM,
(ii) the signal of the lanthanide(III) ion is detected with time-gated luminescence measurement
(iii) The total concentration of A, B, and C is measured based on the signal of lanthanide(III) ion chelated to A, B, and C.

According to this exemplary embodiment the sub-method disclosed above is used for the sample in the first well for examining the total concentration of the sample substances (A, B and C).

### The second well:

The sub-method is as for the first well, but the signal of lanthanide(III) ion is used for quantifying the concentration of B in the sample. Here the lanthanide emission is measured using excitation wavelength specific for compound B.

### The third well:

The sub-method includes measurement of the inherent absorbance of the sample, i.e. the sum of A, B and C. Since only C includes a chromophore, the absorbance is proportional to the concentration of compound C in the sample. If compound C were fluorescent, the detection of signal can be performed based on its fluorescence signal.

After performing the three sub-methods the following information is available.
1st well: total concentration of substances A, B and C
2nd well: concentration of B
3rd well: concentration of C

The results obtained from the three sub-methods are then analyzed by an algorithm which calculates the quantity of each sample substance in the sample. In this particular example, the concentration of substance A can be calculated by subtraction of the concentrations of substance B (2nd well) and C (3rd well) from the total concentration measured (1st well). According to another example the sample includes substances, i.e. organic molecules including two or more chelating groups A, B, and C, and the analysis is performed in a single well. A known amount of lanthanide ion is added, and the signal of the lanthanide(III) ion is detected with time-gated luminescence measurement. The total concentration of A, B, and C is measured based on the signal of lanthanide(III) ion chelated to A, B, and C. Then, the lanthanide emission is correlated to the concentration of compound B using the excitation wavelength specific for compound B. Compound C contains a fluorophore which separately measured to obtain the concentration of compound C.

According to another example the sample includes substances A and B and the analysis is performed in two wells. A known amount of terbium(III) ion is added to the first well and a known amount of europium(III) is added to the second well and the signal of terbium and europium is measured based on the signal of terbium and europium chelated to A and B.

The analysis of the organic polymers according to the method of the present technology is quantitative. Typically, a standard curve or standard point is first prepared and then the concentration of the known sample substance is calculated using a standard curve or a standard point. Alternatively the instrument has been pre-calibrated to support the quantification.

The analysis of the organic polymer according to the method of the present technology is performed in a detection vessel. The vessel may be e.g. a well, a part of a fluidic device or a cuvette. According to one embodiment the polymers are polymers such as sulfonated polycarboxylates, i.e. copolymers comprising allylsulfonate- and maleic anhydride based monomers in 50/50 molar ratio. The molecular weight of the copolymers are typically 1500 and 12 000 Da.

In the present technology, the other sub-methods can be chosen as required based on the nature of the organic molecules in the sample. Exemplary methods of detection are luminescence, fluorescence, absorbance, optical rotation and time-resolved luminescence.

It is known that at high concentration of sample substance such as ionic organic polymers and lanthanide ion, ionic substances readily precipitate. Thus the concentration of the sample substance and the lanthanide ion must be low enough to achieve an optically sufficiently clear solution for measurement.

According to the method disclosed herein, the lanthanide ion concentration must be below 10 mM, preferably between 0.0001 mM and 10 mM, more preferably between 0.001 mM to 10 mM, even more preferably between about 0.1 mM and 0.01 mM and most preferably around 0.01 mM. The concentration of the organic polymer in the sample must be below 10% by weight.

The method of invention is non-specific. Non-specificity should be understood that no specific binders such as high-affinity (> 1·10⁷ M⁻¹) antibodies are used and the interaction of the lanthanide ion with the sample has no pre-determined binding site. Other ions such as cations may bind to the sample as the sample has no pre-determined binding property with the lanthanide ion.

In some applications, the sample includes molecules that have to be removed or diluted prior to analysis. According to this embodiment the sample is subjected to pretreatment step prior to admixing with the lanthanide(III) ion. Exemplary pretreatment methods are size exclusion chromatography, filtration and dilution with an appropriate solution. It should be understood that the pretreatment step means removal or dilution of molecules that may disturb the examination of charged molecules of interest, not isolation of the charged organic polymer e.g. by chromatography.

According to another embodiment the present technology includes a computer program including software modules for determining information indicative for the sample, in order to evaluate the signal derived from the two or more sub-methods. The software modules can be e.g. subroutines of functions implemented with a suitable programming language and with a complier suitable for the programming language and the programmable processor.

A computer program product according to an exemplifying embodiment of the present technology includes a computer readable medium e.g. a compact disc, encoded with a computer program according to an embodiment of the present technology.

A signal according to the exemplary embodiment is encoded to carry information defining a computer program according the embodiment.

### Examples

### Example 1. Measurement of polystyrene sulfonate, PEG 400, PEG 6000, polyethylene imine and BSA with Eu³⁺

The experiment was performed on NUNC Maxisorp microtiter plate (NUNC, Roskilde, Denmark) and 1 mM of EuCl₃ in 250 µL of H₂O. Luminescence signal of Eu³⁺ was monitored at the excitation wavelength of 340 nm and emission wavelength of 615 nm with Victor² V 1420 Multilabel Counter (PerkinElmer, Boston, MA). Results are shown in Figure 3.

### Example 2. Measurement of BSA protein in the presence of a constant concentration of EDTA

The measurement was performed with 5 mM EDTA in H₂O. Luminescence signal of Eu³⁺ was monitored at the excitation wavelength of 285 nm and 319 nm and emission wavelength of 615 nm with Cary Eclipse Spectrofluorometer (Agilent, Santa Clara, CA). Results are shown in Figure 4.

### Example 3. Measurement of EDTA in the presence constant concentration of BSA

The measurement was performed with 50 µM BSA in H₂O. Luminescence signal of Eu³⁺ was monitored at the excitation wavelength of 319 nm and emission wavelength of 615 nm with Cary Eclipse Spectrofluorometer. The results are shown in Figure 5.

### Example 4. Measurement of BSA in the presence of a constant concentration of EDTA

The measurement was performed with 50 µM EDTA. Luminescence signal of Eu³⁺ was monitored at the excitation wavelength of 340 nm and emission wavelength of 615 nm using NUNC Maxisorp microtiter plate and 1 mM of EuCl₃ in 250 µL. BSA and EDTA in water was centrifuged Amicon Ultra-4, cut-off 30kDa wherefrom non-permeate fraction measured. Results are shown in Figure 6.

### Example 5. Measurement of BSA in the presence of three different Eu³⁺ concentrations

Luminescence signal of Eu³⁺ was monitored at the excitation wavelength of 340 nm and emission wavelength of 615 nm with Victor² V 1420 Multilabel Counter using NUNC Maxisorp microtiter plate. EuCl₃ concentration was 0.1 mM, 1 mM and 10 mM. Results are shown in Figure 7.

### Example 6. Measurement of DNA (Mr = 11000) in the presence of different Eu³⁺ concentrations

Luminescence signal of Eu³⁺ was monitored at the excitation wavelength of 340 nm and emission wavelength of 615 nm with Victor² V 1420 Multilabel Counter using NUNC Maxisorp microtiter plate and 0 or 28 µM of DNA in 100 µL of H₂O. Results are shown in Figure 8.

### Example 7. Measurement of DNA (Mr = 11000) labeled with Eu chelate, non-labeled DNA (the same strand) without any label, and 50% labeled DNA + 50% non-labeled DNA using terbium ion

Luminescence signal of Tb³⁺ was monitored at the excitation wavelength of 340 nm and emission wavelength of 545 nm with Victor² V 1420 Multilabel Counter using NUNC Maxisorp microtiter plate and 1 mM of TbCl₃ in 100 µL of H₂O. The total concentration of DNA was measured (labeled DNA + non-labeled DNA). Results are shown in Figure 9.

### Example 8. Measurement of DNA (Mr = 11000) labeled with Eu chelate, non-labeled DNA (the same strand) without any label, and 50% labeled DNA + 50% non-labeled DNA.

Luminescence signal of the Eu chelate was monitored at the excitation wavelength of 340 nm and emission wavelength of 615 nm with Victor² V 1420 Multilabel Counter using NUNC Maxisorp microtiter plate and under the same condition as in the experiment above after diluting the sample 80-fold to H₂O. Concentration of labeled DNA was measured. Results are shown in Figure 10.

### Example 9. Measurement of BSA labeled with FITC and BSA labeled with FITC in the presence of 2 µM of DNA (Mr = 11000) using europium ion.

Luminescence signal of Eu³⁺ was monitored at the excitation wavelength of 340 nm and emission wavelength of 615 nm with Victor² V 1420 Multilabel Counter using NUNC Maxisorp microtiter plate and 1 mM of EuCl₃ in 100 µL of H₂O. The total concentration of BSA-FITC and DNA was measured. Results are shown in Figure 11.

### Example 10. Measurement of BSA labeled with FITC and BSA labeled with FITC in the presence of 2 µM of DNA (Mr = 11000) (square) using fluorescein fluorescence detection.

Luminescence signal of fluorescein was monitored at the excitation wavelength of 485 nm and emission wavelength of 535 nm with Victor² V 1420 Multilabel Counter using NUNC Maxisorp microtiter plate in 100 µL of H₂O. Concentration of BSA-FITC is measured. Results are shown in Figure 12.

### Example 11. Measurement of sulfonated polymers

This example employs the following polymers:
P1: Sulfonated polycarboxylate
P2: Sulfonated polycarboxylate with a fluorescent moiety.

The UV-absorption of the aromatic fluorescent tag was used to transfer excitation energy to the Eu³⁺ ion while chelated by the polymer.

Samples were prepared where the concentrations of P1 varied between 0-30 ppm whereas the concentration of P2 varied between 0-80 ppm.

Total concentration of a mixture of scale inhibitor polymers was determined by adding 2.5 ml of sample in brine (NaCl 35.03 g/l, CaCl₂·2H₂O 2.24 g/l, MgCl₂·6H₂O 1.46 g/l, KCl 0.21 g/l and BaCl₂·2H₂O) to a polystyrene cuvette containing HEPES-NaOH pH 7.4 (25 µl 500 mM) and EuCl₃ (25 µl of 1 mM). After mixing the luminescence signal from Eu³⁺ was monitored at excitation wavelength of 395 nm and emission wavelength of 615 nm with a spectrofluorometer (Aqsens, Espoo, Finland). Standard curve was drawn for the total polymer measurement using a subset of the data and total concentrations were calculated for rest of the data set. The raw data with a standard curve is presented in figure 13.

To determine the concentration of P2 a method with higher sensitivity towards P2 was used. EuCl₃ (50 µl, 500 mM) was placed to UV-transparent cuvette, sample in brine (2.2 ml) was added on top and finally ASP-NaOH pH 9.3 (250 µl) was added. After mixing the luminescence signal from Eu³⁺ was monitored at excitation wavelength of 228 nm and emission wavelength of 615 nm with a spectrofluorometer (Aqsens, Espoo, Finland). The raw data with a standard curve is presented in figure 14.

The raw measurement results from P2 specific method and the calculated total concentrations were divided to training and validation set. The validation set was fed to a trained K-nearest k-Nearest Neighbors regression algorithm to predict the concentration of P2 at low ppm level (< 20 ppm). At higher concentration levels generalized linear model was used to predict the P2 concentration. The concentration of P1 was calculated by subtracting the determined P2 values from total concentrations. Figure 15 illustrates the prediction accuracy of P2 concentration from the validation set. Figure 16 illustrates the prediction accuracy of P1 concentration from the validation set.

It was also observed that quenching of lanthanide chelate luminescence by heavy metals can be utilized when examining samples including sample substances. In the present technology addition of the sample to a mixture of heavy metal ion and a luminescent lanthanide(III) chelate decreases the quenching and gives rise to signal enhancement. According to one aspect the embodiment includes a method for examining a sample comprising two or more sample substances, wherein the method is performed in a plurality of wells. According to this embodiment, the method includes two or more sub methods, wherein the first sub method is according to any of claims 1-7, and wherein one of the sub methods is preferably a second sub method including
- admixing the sample and lanthanide(III) ion, wherein the sample includes an organic molecule to be analyzed, and wherein the total concentration of the organic molecules in the sample is below 10% by weight and the concentration of the lanthanide(III) ion is below 10 mM, preferably from about 10 mM to about 0.001 mM, and wherein the organic molecule comprises two or more chelating groups selected from carboxylates, carboxamides, amines, sulfonates, phosphates and phosphonates, -detecting signal of the lanthanide(III) ion with time-gated luminescence measurement, and
- calculating the quantity of the two or more sample substances in the sample using the signals obtainable by the sub methods.

Other sub-methods can be chosen as required based on the nature of the sample substance in the sample. Exemplary methods of detection are luminescence, fluorescence, absorbance, optical rotation and time-resolved luminescence.

As defined herein a lanthanide chelate is a chemical compound in a form of a heterocyclic ring containing a lanthanide ion attached by coordinate bonds to at least two non-metal ions.

The organic molecule of the embodiment described above includes two or more groups selected from carboxylates, carboxamides, amines, sulfonates, phosphates and phosphonates are able to chelate with a lanthanide(III) ion. The amines can be primary, secondary or tertiary, and the phosphates can be primary or secondary. Accordingly, the chelating groups are the following
- carboxylate (COO⁻),
- sulfonate (-SO₃-)
- carboxamide (-CONH-,-NHCO-, -CONH₂)
- phosphate , wherein R is alkyl
- phosphonate , wherein R is alkyl
and amine.

The net charge of the sample substance including chelating groups may change as a function of pH.

In some applications, the sample includes molecules that have to be removed or diluted prior to analysis. According to this embodiment the sample is subjected to pretreatment step prior to admixing with the lanthanide(III) chelate. Exemplary pretreatment methods are size exclusion chromatography, filtration and dilution with an appropriate solution. It should be understood that the pretreatment step means preferably removal or dilution of molecules that may disturb the examination of charged molecules of interest, not isolation of the charged organic polymer e.g. by chromatography.
the present technology may also include a computer program including software modules for determining information indicative for the sample, in order to evaluate the signal derived from the method. The software modules can be e.g. subroutines of functions implemented with a suitable programming language and with a complier suitable for the programming language and the programmable processor.

A computer program product according to an exemplifying embodiment includes a computer readable medium e.g. a compact disc, encoded with a computer program according to an embodiment of the present technology.

A signal according to the exemplary embodiment is encoded to carry information defining a computer program according the embodiment.

The specific examples provided in the description given above should not be construed as limiting the scope and/or the applicability of the appended claims. It should also be understood that the number of organic compounds quantified and the number of sub-methods used in the method of the present technology is not limited.

## Claims

1. A method for examining a sample containing two or more sample substances comprising one or more organic polymers, wherein at least one organic polymer comprises two or more chelating groups selected from carboxylates, amines, sulfonates, carboxamides, phosphates and phosphonates, the method comprising two or more sub-methods for detecting signals, wherein the sub-methods are selected from fluorescence measurement, luminescence measurement, time-resolved luminescence measurement, and absorbance measurement, wherein at least one of the sub-methods comprises a time resolved luminescence measurement comprising
(i) admixing the sample and lanthanide(III) ion in the form of lanthanide(III) salt, wherein the total concentration of the sample substances in the sample is below 10% by weight and the concentration of the lanthanide(III) ion is ≤ 10 mM,
(ii) detecting signal of the lanthanide(III) ion with time-gated luminescence measurement, and
(iii) calculating the quantity of one or more sample substances using the signal obtainable by the sub-method,
and calculating the quantity of the two or more sample substances based on the signals obtainable by the two or more sub-methods.

2. The method according to claim 1, wherein the lanthanide(III) salt is lanthanide(III) halide.

3. The method according to claim 2, wherein the lanthanide salt is selected from europium(III) chloride and terbium(III) chloride.

4. The method according any of claims 1-3, wherein at least one of the organic polymers examined does not comprise an aromatic group that is able to absorb excitation energy and transfer it essentially to the lanthanide(III) ion.

5. The method according to any of claims 1-4, wherein the method comprises introducing the sample through pretreatment system prior to admixing with the lanthanide(III) ion.

6. The method according to claim 5, wherein the pre-treatment system is selected from dilution, size exclusion chromatography and filtration.

7. The method according to any of claims 1-6 wherein the lanthanide ion concentration is 0.001 mM - 10 mM.

8. The method according to any of claims 1-6 wherein the lanthanide ion concentration is 0.0001 mM-1 mM.

9. The method according to any of claims 1-6 wherein the lanthanide ion concentration is 0.1 mM - 0.01 mM.

10. The method according to any of claims 1-9, wherein one of the sub-methods is a time resolved luminescence measurement comprising excitation of the lanthanide(III) ion at a first excitation wavelength and one sub-method is a time resolved luminescence measurement comprising excitation of the lanthanide(III) ion at a second excitation wavelength.

11. The method according to claim 10, wherein difference of the first excitation wavelength and the second excitation wavelength is more than 10 nm, preferably more than 20 nm, most preferably over 50 nm.

12. A computer program product including computer executable instructions for controlling a programmable processor to examine a sample wherein the program is adapted to evaluate the data obtainable by a method according to any of claims 1-11.

13. A computer readable medium comprising instructions stored thereon which, when executed by a computer, cause the computer to evaluate the data obtainable by a method according to any of claims 1-11.

## Patentansprüche

1. Verfahren zum Untersuchen einer Probe, die zwei oder mehr Probensubstanzen enthält, umfassend ein oder mehrere organische Polymere, wobei mindestens ein organisches Polymer zwei oder mehr Chelatbildnergruppen ausgewählt aus Carboxylaten, Aminen, Sulfonaten, Carboxamiden, Phosphaten und Phosphonaten umfasst, wobei das Verfahren zwei oder mehr untergeordnete Verfahren zum Detektieren von Signalen umfasst, wobei die untergeordneten Verfahren ausgewählt sind aus Fluoreszenzmessung, Lumineszenzmessung, zeitaufgelöster Lumineszenzmessung und Extinktionsmessung, wobei mindestens eines der untergeordneten Verfahren eine zeitaufgelöste Lumineszenzmessung umfasst, welche umfasst:
(i) Mischen von Probe und Lanthanid(III)ion in Form von Lanthanid(III)salz, wobei die Gesamtkonzentration der Probensubstanzen in der Probe unter 10 Gew.% liegt und die Konzentration des Lanthanid(III)ions ≤ 10 mM beträgt,
(ii) Detektieren des Signals des Lanthanid(III)-ions mit zeitfenstergesteuerter Lumineszenzmessung, und
(iii) Berechnen der Menge von einer oder mehreren Probensubstanzen unter Verwendung des Signals, das durch das untergeordnete Verfahren erhältlich ist,
und Berechnen der Menge der zwei oder mehr Probensubstanzen basierend auf den Signalen, die durch die zwei oder mehr untergeordneten Verfahren erhältlich sind.

2. Verfahren nach Anspruch 1, wobei das Lanthanid-(III)salz Lanthanid(III)halogenid ist.

3. Verfahren nach Anspruch 2, wobei das Lanthanidsalz ausgewählt ist aus Europium(III)chlorid und Terbium(III)chlorid.

4. Verfahren nach einem der Ansprüche 1 bis 3, wobei mindestens eines der untersuchten organischen Polymere keine aromatische Gruppe umfasst, die in der Lage ist, Anregungsenergie zu absorbieren und diese im Wesentlichen auf das Lanthanid(III)ion zu übertragen.

5. Verfahren nach einem der Ansprüche 1 bis 4, wobei das Verfahren Einbringen der Probe durch ein Vorbehandlungssystem umfasst, bevor sie mit dem Lanthanid(III)ion gemischt wird.

6. Verfahren nach Anspruch 5, wobei das Vorbehandlungssystem ausgewählt ist aus Verdünnung, Größenausschlusschromatographie und Filtration.

7. Verfahren nach einem der Ansprüche 1 bis 6, wobei die Lanthanidionenkozentration 0,001 mM bis 10 mM beträgt.

8. Verfahren nach einem der Ansprüche 1 bis 6, wobei die Lanthanidionenkozentration 0,0001 mM bis 1 mM beträgt.

9. Verfahren nach einem der Ansprüche 1 bis 6, wobei die Lanthanidionenkozentration 0,1 mM bis 0,01 mM beträgt.

10. Verfahren nach einem der Ansprüche 1 bis 9, wobei eines der untergeordneten Verfahren eine zeitaufgelöste Lumineszenzmessung ist, die Anregung des Lanthanid(III)ions mit einer ersten Anregungswellenlänge umfasst, und ein untergeordnetes Verfahren eine zeitaufgelöste Lumineszenzmessung ist, die Anregung des Lanthanid(III)ions mit einer zweiten Anregungswellenlänge umfasst.

11. Verfahren nach Anspruch 10, wobei die Differenz zwischen der ersten Anregungswellenlänge und der zweiten Anregungswellenlänge mehr als 10 nm, vorzugsweise mehr als 20 nm, am meisten bevorzugt mehr als 50 nm beträgt.

12. Computerprogrammprodukt, das computerausführbare Anweisungen zum Steuern eines programmierbaren Prozessors einschließt, um eine Probe zu untersuchen, wobei das Programm vorgesehen ist, um die Daten auszuwerten, die nach einem Verfahren gemäß einem der Ansprüche 1 bis 11 erhältlich sind.

13. Computerlesbares Medium, das darauf gespeicherte Anweisungen umfasst, die bei Ausführung durch einen Computer herbeiführen, dass der Computer die Daten auswertet, die nach einem Verfahren gemäß einem der Ansprüche 1 bis 11 erhältlich sind.

## Revendications

1. Procédé d'examen d'un échantillon contenant deux ou plus de deux substances d'échantillon comprenant un ou plusieurs polymères organiques, dans lequel au moins un polymère organique comprend deux ou plus de deux groupes chélateurs choisis parmi des carboxylates, des amines, des sulfonates, des carboxamides, des phosphates et des phosphonates, le procédé comprenant deux ou plus de deux sous-procédés pour détecter des signaux, dans lequel les sous-procédés sont choisis parmi une mesure de fluorescence, une mesure de luminescence, une mesure de luminescence à résolution temporelle, et une mesure d'absorbance, dans lequel au moins l'un des sous-procédés comprend une mesure de luminescence à résolution temporelle comprenant
(i) le mélange de l'échantillon et d'ion de lanthanide(III) sous la forme de sel de lanthanide(III), dans lequel la concentration totale des substances d'échantillon dans l'échantillon est inférieure à 10 % en poids et la concentration de l'ion de lanthanide(III) est ≤ 10 mM,
(ii) la détection du signal de l'ion de lanthanide(III) avec une mesure de luminescence à déclenchement temporel, et
(iii) le calcul de la quantité d'une ou plusieurs substances d'échantillon en utilisant le signal pouvant être obtenu par le sous-procédé,
et le calcul de la quantité des deux ou plus de deux substances d'échantillon sur la base des signaux pouvant être obtenus par les deux ou plus de deux sous-procédés.

2. Procédé selon la revendication 1, dans lequel le sel de lanthanide(III) est un halogénure de lanthanide(III).

3. Procédé selon la revendication 2, dans lequel le sel de lanthanide est choisi parmi le chlorure d'europium(III) et le chlorure de terbium(III).

4. Procédé selon l'une quelconque des revendications 1 à 3, dans lequel au moins un des polymères organiques examinés ne comprend pas un groupe aromatique qui est capable d'absorber une énergie d'excitation et transférer celle-ci essentiellement à l'ion lanthanide(III).

5. Procédé selon l'une quelconque des revendications 1 à 4, le procédé comprenant l'introduction de l'échantillon à travers un système de prétraitement avant mélange avec l'ion lanthanide(III).

6. Procédé selon la revendication 5, dans lequel le système de prétraitement est choisi parmi la dilution, la chromatographie d'exclusion et la filtration.

7. Procédé selon l'une quelconque des revendications 1 à 6, dans lequel la concentration d'ion lanthanide est de 0,001 mM à 10 mM.

8. Procédé selon l'une quelconque des revendications 1 à 6, dans lequel la concentration d'ion lanthanide est de 0,0001 mM à 1 mM.

9. Procédé selon l'une quelconque des revendications 1 à 6, dans lequel la concentration d'ion lanthanide est de 0,1 mM à 0,01 mM.

10. Procédé selon l'une quelconque des revendications 1 à 9, dans lequel l'un des sous-procédés est une mesure de luminescence à résolution temporelle comprenant l'excitation de l'ion lanthanide(III) à une première longueur d'onde d'excitation et un sous-procédé est une mesure de luminescence à résolution temporelle comprenant l'excitation de l'ion lanthanide(III) à une deuxième longueur d'onde d'excitation.

11. Procédé selon la revendication 10, dans lequel la différence entre la première longueur d'onde d'excitation et la deuxième longueur d'onde d'excitation est supérieure à 10 nm, de préférence supérieure à 20 nm, de manière préférée entre toutes supérieure à 50 nm.

12. Produit de programme informatique comprenant des instructions exécutables par ordinateur pour commander un processeur programmable pour examiner un échantillon, dans lequel le programme est adapté pour évaluer les données pouvant être obtenues par un procédé selon l'une quelconque des revendications 1 à 11.

13. Support lisible par ordinateur comprenant des instructions stockées sur celui-ci qui, lorsqu'elles sont exécutées par un ordinateur, amènent l'ordinateur à évaluer les données pouvant être obtenues par un procédé selon l'une quelconque des revendications 1 à 11.
